# EUROPÄISCHE PATENTSCHRIFT

(11) **EP 3 093 645 B1**
(45) Veröffentlichungstag und Bekanntmachung des Hinweises auf die Patenterteilung: **31.07.2019**
(21) Anmeldenummer: 16161972.1
(22) Anmeldetag: 23.03.2016
(51) Int. Cl.: G01N 1/22, G01N 33/00, G01N 25/56

(54) **VORRICHTUNG UND VERFAHREN ZUM UNTERSUCHEN VON SCHICHTMATERIAL AUF VERUNREINIGUNG**
METHOD AND DEVICE FOR INSPECTING COATING MATERIAL FOR CONTAMINATION
DISPOSITIF ET PROCEDE DE RECHERCHE D'IMPURETES DANS UN MATERIAU STRATIFIE

(30) Priorität: 11.05.2015 DE 102015107341
(43) Veröffentlichungstag der Anmeldung: 16.11.2016
(73) Patentinhaber: Airbus Defence and Space GmbH, 85521 Ottobrunn (DE)
(72) Erfinder: Wachinger, Georg, 85521 Ottobrunn (DE); Helwig, Andreas, 85521 Ottobrunn (DE); Meer, Thomas, 85521 Ottobrunn (DE); Geistbeck, Matthias, 85521 Ottobrunn (DE); Friedberger, Alois, 85521 Ottobrunn (DE); Heckner, Sebastian, 85521 Ottobrunn (DE)
(74) Vertreter: Isarpatent

(56) Entgegenhaltungen:
- EP-A1- 0 897 108
- DE-A1-102011 102 055
- US-A1- 2002 148 974

## Beschreibung

Die vorliegende Erfindung betrifft eine Vorrichtung und ein Verfahren zur Untersuchung von Schichtmaterial auf Verunreinigung.

Während der Herstellung und des Einsatzes von Schichtmaterial, insbesondere von Elementen aus Faserverbundkunststoffen (wie beispielsweise kohlenstofffaserverstärktem Kunststoff) können unterschiedliche Stoffe, die mit dem Schichtmaterial in Berührung kommen (wie z.B. Feuchte, Reinigungsmittel, Frostschutz- oder Enteisungsmittel, Betriebsstoffe usw.) in das Schichtmaterial hineindiffundieren.

Insbesondere können vorimprägnierte, ungehärtete Faser - Matrix - Matten (sogenannte "Prepregs") während Versand, Lagerung und/oder Handhabung in der Fertigung auf diese Weise Kontaminationen aufnehmen, beispielsweise in Form von Feuchtigkeit. Die Aufnahme ist abhängig von der Matrixzusammensetzung, den Lagerbedingungen und den Klimabedingungen in der Fertigung.

Aus derart verunreinigten Materialien hergestellte Laminate können eine nachteilige ungleichmäßige Struktur aufweisen, die beispielsweise Lufteinschlüsse umfasst, und sie können für nachfolgende Verarbeitungsschritte wie insbesondere Verklebungen ungeeignet sein.

Im Rahmen von Gegenmaßnahmen können verschiedene Kontaminationen beseitigt oder zumindest reduziert werden. Insbesondere werden beispielsweise standardisierte, z.T. zeitintensive Zyklen angewandt, ohne dass jedoch vorab erfasst würde, ob und in welchem Umfang eine Maßnahme in einem konkreten Fall tatsächlich erforderlich ist. Dabei werden häufig sogar negative Einflüsse der Maßnahme auf das spätere Bauteil, die sich bei bestimmten Temperaturen und Einwirkzeiten auf das ungehärtete Material ergeben können (beispielsweise nachteilige Viskositäten oder Anreaktionszustände) in Kauf genommen.

Es ist daher von Bedeutung, eine Kontaminierung des verwendeten Materials erfassen zu können.

Aus der Druckschrift DE 10 2011 102 055 A1 ist eine Vorrichtung bekannt, mittels der ein gehärtetes Faserverbundbauteil auf das Vorhandensein einer Mehrzahl bestimmter Kontaminationsstoffe geprüft werden kann. Diese Vorrichtung umfasst insbesondere eine Oberflächenerwärmungsvorrichtung und eine Sensoranordnung zur Erfassung von Kontaminationsstoffen, die von der erwärmten Oberfläche des Faserverbundteils desorbiert wurden. Als derartige Oberflächenerwärmungsvorrichtungen sind dabei ein auf dem Substrat aufliegender Heizstempel sowie eine Halogenlampe offenbart.

Die Druckschrift US 2002/0148974 A1 beschreibt ein Desorptionssystem mit einer Heizkammer, welche von einer Infrarot-Wärmequelle bestrahlt werden kann. Auf einer der Infrarot-Wärmequelle abgewandten Seite der Heizkammer kann ein Wafer angeordnet sein, dessen Temperatur mit einem Thermoelement gemessen werden kann.

Ein weiterer Ansatz ist es, eine Feuchteverunreinigung eines Probenmaterials mit Hilfe eines Infrarot-Sensors zu detektieren; dies erlaubt einen Verzicht auf eine Erwärmung des untersuchten Materials. Ein Nachteil einer derartigen Bestimmung ist jedoch, dass lediglich eine Kontamination einer obersten Matrixharzschicht (< 100 µm von der Oberfläche) erfasst wird, weil die Infrarotstrahlung auf der darunter liegenden Faserlage reflektiert wird. Die jeweils erfasste Feuchtigkeit hängt deshalb stark von einer Feuchtigkeit der Umgebung ab, was die Genauigkeit der Messung beeinträchtigt. Darüber hinaus muss bei diesem Vorgehen die diffuse Infrarot-Rückstreuung durch die Faserlagen mit berücksichtigt werden, was ebenfalls eine genaue Bestimmung der Verunreinigung erschwert.

Die vorliegende Erfindung hat daher die Aufgabe, ein verbesserte Technik bereitzustellen, mit der Kontaminationen insbesondere auch in ungehärteten Faser - Matrix - Verbundwerkstoffen erfasst werden können.

Die Aufgabe wird gelöst durch eine Vorrichtung gemäß Anspruch 1 und ein Verfahren gemäß Anspruch 7. Bevorzugte Ausführungsformen sind in den Unteransprüchen offenbart.

Eine erfindungsgemäße Vorrichtung eignet sich zur Untersuchung von Schichtmaterial auf Verunreinigungen. Sie umfasst eine Heizvorrichtung zum Erwärmen einer Probenfläche des Schichtmaterials und eine Detektoreinrichtung zum Erfassen mindestens eines aus dem Schichtmaterial in ein Messvolumen desorbierten Kontaminationsstoffes. Die erfindungsgemäße Vorrichtung umfasst weiterhin eine Messglocke, die dazu eingerichtet ist, um das Schichtmaterial herum oder an dem Schichtmaterial anliegend das Messvolumen auszubilden, das an die Probenfläche angrenzt. Die Messglocke weist ein energiedurchlässiges Fenster auf. Die Detektoreinrichtung ist innerhalb der Messglocke und des Messvolumens angeordnet. Die Heizvorrichtung umfasst dabei mindestens ein Infrarot-Erwärmungselement, welches dazu eingerichtet, die Probenfläche durch das Fenster hindurch zu bestrahlen.

Ein erfindungsgemäßes Verfahren eignet sich zur Untersuchung von Schichtmaterial auf Verunreinigungen. Es umfasst ein Erwärmen einer Probenfläche des Schichtmaterials mit einem Infrarot-Erwärmungselement und ein Erfassen mindestens eines aus dem Schichtmaterial in ein Messvolumen desorbierten Kontaminationsstoffes. Das Erwärmen erfolgt mittels eines Infrarot-Erwärmungselements. Die Probenfläche wird unter einer Messglocke erwärmt, die um das Schichtmaterial herum oder an das Schichtmaterial anliegend das Messvolumen ausbildet. Die Messglocke weist ein energiedurchlässiges Fenster auf und das Infrarot-Erwärmungselement bestrahlt die Probenfläche durch das Fenster hindurch. Die Detektoreinrichtung ist innerhalb der Messglocke und des Messvolumens angeordnet.

Das Schichtmaterial kann mehrere Schichten aufweisen oder dazu eingerichtet sein, für die Herstellung eines Laminats geschichtet zu werden. Insbesondere kann das Schichtmaterial eine vorimprägnierte, ungehärtete Faser-Matrix-Matte (ein "Prepreg") umfassen, die dazu eingerichtet ist, zur Herstellung eines Laminats geschichtet und danach (z.B. in einem Autoklaven) ausgehärtet zu werden.

Das Infrarot-Erwärmungselement kann insbesondere einen Infrarotstrahler umfassen. Die erfindungsgemäße Erwärmung mit Hilfe des (vorzugsweise regelbaren) Infrarot-Erwärmungselements ermöglicht ein kontaktfreies Erwärmen (insbesondere) eines ungehärteten, nicht formstabilen Verbundwerkstoffs (wie beispielsweise einer vorimprägnierten, ungehärteten Faser - Matrix - Matte), ohne dass eine Umgebungsluft zu einem nachteiligen Grade miterhitzt würde. Auf diese Weise kann zwar einerseits eine Desorption von Kontaminationsstoffen thermisch aktiviert werden, eine aus erhitzter Umgebungsluft resultierende Beeinträchtigung der Viskosität und des Anreaktionszustandes des ungehärteten Materials kann jedoch vermieden oder zumindest minimiert werden. Eine erfindungsgemäße Vorrichtung und ein erfindungsgemäßes Verfahren eignen sich daher zur Kontaminationsbestimmung auch ungehärteter Verbundstoffe und ermöglichen eine vorteilhafte Einstellung von Parametern gegebenenfalls notwendiger Gegenmaßnahmen. Auf diese Weise können Fehlstellen im Laminat und auf der Oberfläche verhindert und so eine Laminat-, Klebe- und Oberflächenqualität von aus dem Material hergestellten Bauteilen optimiert werden.

Insbesondere ermöglicht die vorliegende Erfindung, eine gegebenenfalls vorliegende Verunreinigung (z.B. einen realen Feuchtegehalt) einer vorimprägnierten Faser- Matrix-Matte (eines "Prepregs") während eines Legeprozesses zur Fertigung eines Laminats zu bestimmen, und/oder entsprechend begleitend zur Fertigung hergestellte Begleitproben entsprechend zu untersuchen.

Das Anwendungsgebiet einer erfindungsgemäßen Vorrichtung und eines erfindungsgemäßen Verfahrens ist jedoch nicht auf ungehärtete Verbundstoffe beschränkt, sondern beide können auch für eine Untersuchung anderer Materialien, insbesondere ausgehärteter Faserverbundstoffe (z. B. vor einer Weiterverarbeitung durch Kleben, Lackieren o.ä.) herangezogen werden. Die vorliegende Erfindung bietet somit weiterhin den Vorteil einer flexiblen Einsetzbarkeit.

Die Probenfläche kann jeweils eine Gesamtoberfläche des Schichtmaterials oder ein lokaler Oberflächenabschnitt des Schichtmaterials sein; an ihr erfolgt eine Untersuchung des Schichtmaterials.

Gemäß einer bevorzugten Ausführungsform einer erfindungsgemäßen Vorrichtung umfasst die Detektoreinheit einen oder mehrere Sensoren; analog erfolgt das Erfassen gemäß einem erfindungsgemäßen Verfahren vorzugsweise mittels einer solchen Detektoreinheit. Insbesondere kann die Detektoreinheit beispielsweise mindestens einen Feuchtesensor, Metalloxidsensor, nichtdispersiven Infrarotsensor, mindestens ein lonenmobilitätsspektrometer (IMS); und/oder mindestens einen Gas-Chromatographiesensor (der z.B. ein lonenmobilitätsspektrometer als Detektor aufweisen kann) umfassen. Das Messen kann ein Bestimmen einer absoluten oder relativen Menge des mindestens einen Kontaminationsstoffs im Messvolumen umfassen.

Vorzugsweise umfasst die Detektoreinheit mehrere Sensoren, die dazu eingerichtet sind, jeweils einen anderen einer vorgegebenen Auswahl an Kontaminationsstoffen zu erfassen. Besonders bevorzugt ist eine Ausführungsform der erfindungsgemäßen Vorrichtung, bei der die Detektoreinheit dazu eingerichtet ist, den mindestens einen aus dem Schichtmaterial desorbierten Kontaminationsstoff zu quantifizieren. Analog umfasst eine besonders bevorzugte Variante des erfindungsgemäßen Verfahrens ein Quantifizieren des mindestens einen aus dem Schichtmaterial desorbierten Kontaminationsstoffs.

Die somit erhaltenen Mengenwerte für den Kontaminationsstoff im Schichtmaterial können insbesondere dazu dienen, geeignete Parameter mindestens einer Gegenmaßnahmen zu bestimmen und einzustellen, mit der eine erfasste und quantifizierte Verunreinigung beseitigt oder reduziert werden kann. Derartige Parameter können beispielsweise eine Dauer der Maßnahme oder eine Temperatur sein, der das Schichtmaterial im Rahmen der Gegenmaßnahme ausgesetzt wird.

Bevorzugt ist eine Ausführungsvariante der erfindungsgemäßen Vorrichtung, bei der die Heizvorrichtung dazu eingerichtet ist, eine Teilfläche der Probenfläche simultan zu bestrahlen, wobei die Teilfläche eine Größe von mindestens 100 cm<2>, bevorzugter von mindestens 625 cm<2>oder sogar mindestens 900 cm<2>hat; analog umfasst das Erwärmen gemäß einer bevorzugten Ausführungsform eines erfindungsgemäßen Verfahrens ein simultanes Bestrahlen einer entsprechenden großen Teilfläche der Probenfläche. Die bestrahlte Teilfläche kann dabei beispielsweise im Wesentlichen rechteckig (z.B. im Wesentlichen quadratisch) oder im Wesentlichen eine Kreisfläche sein.

Das Erwärmen der Probenfläche kann somit flächig über eine entsprechend große Ausdehnung erfolgen. Dadurch können nachteilige lokale Verformungen von nicht formstabilem, ungehärtetem Material vermieden werden, die aus einer nur lokalen Erwärmung (z.B. auf einer Fläche von nur etwa 3cm^{∗}3cm) aufgrund daraus resultierender hoher Temperaturgradienten auftreten können.

Vorzugsweise ist die Heizvorrichtung mit dem Infrarot-Erwärmungselement Teil eines mobilen, tragbaren Handgeräts. Besonders bevorzugt ist eine Ausführungsform, bei der die gesamte erfindungsgemäße Vorrichtung Teil eines derartigen mobilen, tragbaren Handgeräts ist oder zumindest tragbare, mobile Komponenten aufweist. Insbesondere für eine stichprobenartige Untersuchung des Materials an mehreren Stellen kann eine derartige Vorrichtung leicht an dem Schichtenmaterial umpositioniert werden, wohingegen das Schichtenmaterial an seiner Position belassen werden kann. Dies ist insbesondere bei großflächigem, nicht formstabilem Schichtenmaterial vorteilhaft, das schwierig zu bewegen ist.

Die erfindungsgemäße Vorrichtung umfasst eine Messglocke, die dazu eingerichtet ist, um das Schichtmaterial herum oder an dem Schichtmaterial anliegend ein Messvolumen auszubilden, das an die Probenfläche angrenzt; die Detektoreinrichtung erfasst dabei vorzugsweise den mindestens einen Kontaminationsstoff im Messvolumen.

Gemäß einem erfindungsgemäßen Verfahren wird analog die Probenfläche vorzugsweise unter einer derartigen Messglocke erwärmt und der mindestens eine Kontaminationsstoff im Messvolumen darin erfasst.

Die Probenfläche bildet dabei zusammen mit der Messglocke und ggf. mit einer weiteren Fläche (beispielsweise einer Unterlage) eine Begrenzung des (um- oder anliegenden) Messvolumens. Beispielsweise kann die Messglocke in einer beliebigen Richtung auf die Probenfläche des Schichtmaterials oder eine Unterlage gestülpt sein, oder sie kann das Schichtmaterial ganz umschließen. Insbesondere ist der präpositionale Ausdruck "unter einer Messglocke" nicht als auf eine vertikale Ausrichtung hinweisend zu interpretieren.

Das Messvolumen kann einen beliebig geformten geometrischen Raum ausbilden (wie beispielsweise eine Kugel, eine Halbkugel, einen Zylinder, einen Kegelstumpf oder einen Quader, um nur einige zu nennen). Insbesondere ist der Begriff der das Messvolumen definierenden "Messglocke" nicht als Beschränkung der Geometrie des Messvolumens zu verstehen.

Bei derartigen, eine Messglocke umfassenden Ausführungsformen kann insbesondere eine Genauigkeit verbessert werden, mit welcher der mindestens eine aus dem Schichtmaterial desorbierte Kontaminationsstoff erfasst wird, weil dieser Stoff mindestens zu einem Teil im Messvolumen aufgefangen wird und sich somit höchstens begrenzt in der Umgebung verteilt.

Darüber hinaus kann eine derartige Messglocke ein Austreten von gesundheitsschädlichem und/oder schlecht riechendem Gas verhindern, das sich durch die thermisch aktivierte Desorption entwickelt haben kann.

Gemäß einer bevorzugten Ausführungsform der vorliegenden Erfindung ist die Messglocke mindestens ein Teil eines tragbaren Handgeräts. Insbesondere kann sie vorzugsweise einen Durchmesser von mindestens 10 cm und/oder von maximal 50 cm, bevorzugter maximal 20 cm haben, und/oder eine Masse von höchstens 5 kg, bevorzugter von höchstens 1 kg.

Eine derartige Messglocke kann somit leicht umpositioniert werden, was beispielsweise ein stichprobenartiges Untersuchen des Schichtmaterials an mehreren verschiedenen Probenflächen erleichtert.

Die Messglocke weist ein energiedurchlässiges Fenster auf und das Infrarot-Erwärmungselement ist dazu eingerichtet, die Probenfläche durch das Fenster hindurch zu bestrahlen. Auf diese Weise kann durch eine geeignete Entfernung des Infrarot-Erwärmungselements von der Probenfläche eine breite Streuung des Infrarotlichts erreicht werden, ohne dass das Messvolumen entsprechend groß gewählt werden muss, was eine Genauigkeit der Erfassung von Kontaminationsstoff im Messvolumen mindern würde.

Alternativ oder zusätzlich kann die Vorrichtung eine Spülvorrichtung umfassen, die dazu eingerichtet ist, das Messvolumen mit Gas zu spülen; ein erfindungsgemäßes Verfahren umfasst analog vorzugsweise ein derartiges Spülen.

Durch das Spülen wird im Messvolumen befindliches Gas ausgetauscht. Vorzugsweise wird dabei solches Gas in das Messvolumen geleitet, das vorbekannte Eigenschaften hat, insbesondere, dessen Kontaminierung mit dem mindestens einen Kontaminationsstoff einen vorbekannten Schwellwert nicht überschreitet. Gemäß einer bevorzugten Ausführungsform ist das Gas Luft, vorzugsweise synthetische Luft. Diese bietet die Vorteile, besonders kostengünstig, geruchlos, ungiftig und nicht brennbar zu sein.

Besonders bevorzugt ist eine Ausführungsform, bei der das Erfassen des mindestens einen aus dem Schichtmaterial desorbierten Kontaminationsstoffes mehrmals erfolgt, und zwar vorzugsweise nach und vor einem Spülen des Messvolumens.

Dadurch kann das Erfassen präzisiert werden. Beim Spülen wird nämlich insbesondere Kontaminationsstoff aus dem Messvolumen entfernt, der gegebenenfalls zuvor von der oder durch die Probenfläche des Schichtmaterials in das Messvolumen desorbiert wurde. Das Spülen verhindert somit eine Sättigung des Messvolumens mit dem mindestens einen Kontaminationsstoff. Auf diese Weise wird eine fortgesetzte Desorption des mindestens einen Kontaminationsstoffes in das Messvolumen ermöglicht und damit ein genaues Erfassen einer Verunreinigung des Schichtmaterials verbessert.

Im Folgenden werden bevorzugte Ausführungsbeispiele der Erfindung anhand einer Zeichnung näher erläutert. Es versteht sich, dass einzelne Elemente und Komponenten auch anders kombiniert werden können als dargestellt.

Es zeigt schematisch:

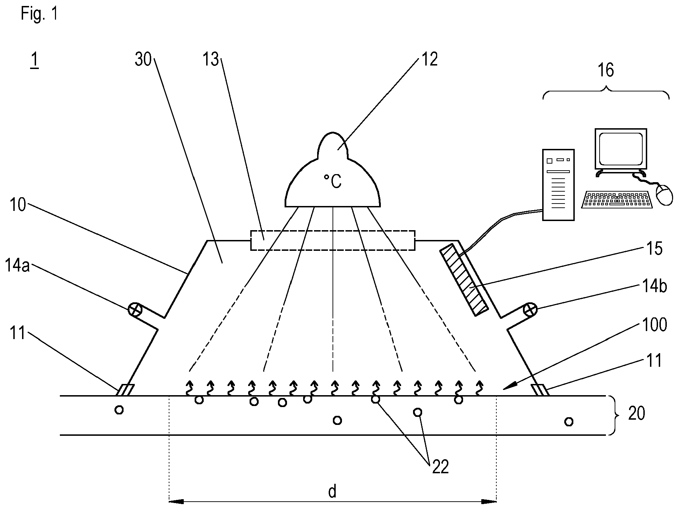

Figur 1 : eine Vorrichtung gemäß einer beispielhaften Ausführungsform der vorliegenden Erfindung.

In Figur 1 ist (nicht maßstabsgetreu) eine Anordnung schematisch dargestellt, die eine Ausführungsform einer erfindungsgemäßen Vorrichtung 1 umfasst.

Die Vorrichtung umfasst eine Messglocke 10, die an einem Schichtmaterial 20 anliegend ein Messvolumen 30 ausbildet. Insbesondere wird das Messvolumen 30 von der Messglocke und einer Probenfläche 100, die Teil einer Oberfläche des Schichtmaterials 20 ist, eingeschlossen. Insbesondere grenzt die Probenfläche 100 an das Messvolumen 30 an.

Die Messglocke 10 weist an ihrem Rand eine Abdichtung 11 auf, die das Messvolumen an einem Übergang zwischen der Messglocke 10 und der Probenfläche 100 abdichtet.

Das Schichtmaterial 20 kann beispielsweise ein ungehärtetes Faser-Matrix-Verbundmaterial umfassen. Es enthält in seinem Innern einen Kontaminationsstoff 22.

Die Vorrichtung 1 umfasst weiterhin ein Infrarot-Erwärmungselement 12 als Heizvorrichtung, das dazu eingerichtet ist, die Probenfläche 100 aufzuheizen. Wie schematisch dargestellt ist, wird dabei vom Infrarot-Erwärmungselement 12 eine Teilfläche der Probenfläche bestrahlt, die im gezeigten Querschnitt einen Durchmesser d hat; gemäß einer bevorzugten Ausführungsform ist d ≥ 10cm, noch bevorzugter ist d ≥ 30cm.

Gemäß einer bevorzugten Ausführungsform ist das Infrarot-Erwärmungselement 12 regelbar, flächig und/oder mobil.

Im dargestellten Ausführungsbeispiel ist die Heizvorrichtung 12 außerhalb der Messglocke 10 angeordnet, und sie erwärmt die Probenfläche durch ein in der Messglocke 10 befindliches energiedurchlässiges Fenster 13.

Eine Spüleinheit 14a, 14b der Vorrichtung 1 umfasst einen vorzugsweise regelbaren Gasanschluss 14a und einen Gasauslass 14b und ist dazu eingerichtet, ein vorbestimmtes Gas (beispielsweise synthetische Luft) durch das Messvolumen hindurchzuleiten und dabei zusammen mit dem im Messvolumen befindlichen Gas Kontaminationsstoff aus dem Messvolumen 30 hinauszuspülen.

Die Vorrichtung 1 umfasst ferner eine Detektoreinrichtung 15 (mit einem oder mehreren Sensoren), die dazu eingerichtet ist, eine Kontaminierung des Messvolumens 30 mit (mindestens) dem Kontaminationsstoff 22 qualitativ und/oder quantitativ zu messen. Schließlich umfasst die Vorrichtung 1 eine Rechnereinheit 16, die an die Detektoreinrichtung 15 angeschlossen und dazu eingerichtet ist, der Detektoreinrichtung 15 erfasste Messwerte geeignet auszuwerten. Besonders bevorzugt ist eine Ausführungsform, bei der eine derartige Rechnereinheit auf Grundlage der von der Detektoreinheit erfassten Werte automatisiert einen oder mehrere Parameter einer Gegenmaßnahme bestimmt, die geeignet ist, den mindestens einen Kontaminationsstoff aus dem Schichtmaterial zu entfernen.

In Verwendung der Vorrichtung wird nun die Probenfläche 100 mit Hilfe der Heizvorrichtung 12 erhitzt, was eine Desorption des Kontaminationsstoffes 22 aus einer oberflächennahen Schicht der Probenfläche in das Messvolumen 30 bewirkt, wie durch die Pfeile schematisch dargestellt. Das Sensorsystem 15 misst die daraus resultierende Kontaminierung des Messvolumens, vorzugsweise mehrere Male, und leitet die jeweils gemessenen Werte an die Rechnereinheit 16 weiter.

### Bezugszeichen

- 1: Vorrichtung
- 10: Messglocke
- 11: Abdichtung
- 12: Heizvorrichtung mit Infrarot-Erwärmungselement
- 13: Fenster
- 14a, 14b: Spüleinheit
- 15: Detektoreinrichtung
- 16: Rechnereinheit
- 20: Schichtmaterial
- 22: Kontaminationsstoff
- 30: Messvolumen
- 100: Probenfläche

- d: Durchmesser eines Teilbereichs

## Patentansprüche

1. Vorrichtung (1) zum Untersuchen von Schichtmaterial (20) auf Verunreinigung, wobei die Vorrichtung eine Heizvorrichtung (12) zum Erwärmen einer Probenfläche (100) des Schichtmaterials und eine Detektoreinrichtung (15) zum Erfassen mindestens eines aus dem Schichtmaterial in ein Messvolumen (30) desorbierten Kontaminationsstoffes (22) umfasst,
wobei die Heizvorrichtung mindestens ein Infrarot-Erwärmungselement umfasst, wobei die Vorrichtung eine Messglocke (10) umfasst, die dazu eingerichtet ist, um das Schichtmaterial herum oder an dem Schichtmaterial (20) anliegend das Messvolumen (30) auszubilden, das an die Probenfläche angrenzt, und
wobei die Messglocke (10) ein energiedurchlässiges Fenster (13) aufweist und das Infrarot-Erwärmungselement dazu eingerichtet ist, die Probenfläche (100) durch das Fenster (13) hindurch zu bestrahlen, **dadurch gekennzeichnet, dass** die Detektoreinrichtung (15) innerhalb der Messglocke (10) und des Messvolumens (30) angeordnet ist.

2. Vorrichtung gemäß Anspruch 1, wobei die Heizvorrichtung dazu eingerichtet ist, eine Teilfläche der Probenfläche simultan zu bestrahlen, wobei die Teilfläche eine Größe von mindestens 100 cm², bevorzugter von mindestens 625 cm² oder sogar mindestens 900 cm² hat.

3. Vorrichtung gemäß Anspruch 1 oder 2, wobei die Detektoreinrichtung (15) dazu eingerichtet ist, den mindestens einen aus dem Schichtmaterial desorbierten Kontaminationsstoff zu quantifizieren.

4. Vorrichtung gemäß einem der vorhergehenden Ansprüche, die Teil eines mobilen, tragbaren Handgeräts ist.

5. Vorrichtung gemäß Anspruch 1, die zudem eine Spülvorrichtung (14a, 14b) umfasst, die dazu eingerichtet ist, das Messvolumen mit Gas zu spülen.

6. Vorrichtung gemäß einem der vorhergehenden Ansprüche, wobei das zu untersuchende Schichtmaterial einen ungehärteten Faser - Matrix-Verbundwerkstoff und/oder einen gehärteten Verbundwerkstoff umfasst.

7. Verfahren zum Untersuchen von Schichtmaterial (20) auf Verunreinigung, das ein Erwärmen einer Probenfläche (100) des Schichtmaterials und ein Erfassen mindestens eines aus dem Schichtmaterial in ein Messvolumen (30) desorbierten Kontaminationsstoffes (22) umfasst,
wobei das Erwärmen mittels eines Infrarot-Erwärmungselements erfolgt, und
wobei die Probenfläche (100) unter einer Messglocke (10) erwärmt wird, die um das Schichtmaterial herum oder an das Schichtmaterial anliegend das Messvolumen (30) ausbildet, und die Messglocke (10) ein energiedurchlässiges Fenster (13) aufweist und das Infrarot-Erwärmungselement die Probenfläche (100) durch das Fenster (13) hindurch bestrahlt, wobei die Detektoreinrichtung (15) innerhalb der Messglocke (10) und des Messvolumens (30) angeordnet ist.

8. Verfahren gemäß Anspruch 7, wobei das Erwärmen ein simultanes Bestrahlen einer Teilfläche der Probenfläche (100) umfasst, wobei die Teilfläche eine Größe von mindestens 100 cm², bevorzugter von mindestens 625 cm² oder sogar mindestens 900 cm² hat.

9. Verfahren gemäß Anspruch 7 oder 8, das ein Quantifizieren des mindestens einen aus dem Schichtmaterial desorbierten Kontaminationsstoffs (22) umfasst.

10. Verfahren gemäß einem der Ansprüche 7 bis 9, das zudem ein Spülen des Messvolumens (30) mit Gas umfasst.

## Claims

1. Device (1) for inspecting coating material (20) for contamination, wherein the device comprises a heating device (12) for heating a specimen area (100) of the coating material and a detection installation (15) for detecting at least one contamination substance (22) desorbed from the coating material into a measuring volume (30), wherein the heating device comprises at least one infrared heating element,
wherein the device comprises a measuring bell (10) which is specified for configuring the measuring volume about the coating material or so as to bear on the coating material (20), said measuring volume being contiguous to the specimen area, and wherein the measuring bell (10) has an energy-permeable window (13) and the infrared heating element is specified for irradiating the specimen area (100) through the window (13),
**characterized in that**
the detection installation (15) is disposed within the measuring bell (10) and the measuring volume (30) .

2. Device according to Claim 1, wherein the heating device is specified for simultaneously irradiating a part-area of the specimen area, wherein the part-area has a size of at least 100 cm², more preferably of at least 625 cm², or even at least 900 cm².

3. Device according to Claim 1 or 2, wherein the detection installation (15) is specified for quantifying the at least one contamination substance desorbed from the coating material.

4. Device according to one of the preceding claims, said device being part of a mobile portable handheld apparatus.

5. Device according to Claim 1, said device moreover comprising a purging device (14a, 14b) which is specified for purging the measuring volume with gas.

6. Device according to one of the preceding claims, wherein the coating material to be inspected comprises a non-cured fibre-matrix composite material and/or a cured composite material.

7. Method for inspecting coating material (20) for contamination, said method comprising heating a specimen area (100) of the coating material and detecting at least one contamination substance (22) desorbed from the coating material into a measuring volume (30),
wherein the heating is performed by means of an infrared heating element, and
wherein the specimen area (100) is heated under a measuring bell (10) which configures the measuring volume (30) about the coating material or so as to bear on the coating material, and the measuring bell (10) has an energy-permeable window (13) and the infrared heating element irradiates the specimen area (100) through the window (13), wherein the detection installation (15) is disposed within the measuring bell (10) and the measuring volume (30).

8. Method according to Claim 7, wherein the heating comprises simultaneously irradiating a part-area of the specimen area (100), wherein the part-area has a size of at least 100 cm², more preferably of at least 625 cm², or even at least 900 cm².

9. Method according to Claim 7 or 8, said method comprising quantifying the at least one contamination substance (22) desorbed from the coating material.

10. Method according to one of Claims 7 to 9, said method moreover comprising purging of the measuring volume (30) with gas.

## Revendications

1. Dispositif (1), destiné à examiner une matière stratifiée (20) au niveau des impuretés, le dispositif comprenant un dispositif de chauffage (12), destiné à chauffer une surface échantillon (100) de la matière stratifiée et un système détecteur (15), destiné à détecter au moins un contaminant (22) désorbé de la matière stratifiée dans un volume de mesure (30),
le dispositif de chauffage comprenant au moins un élément chauffant par infra-rouges, le dispositif comprenant une cloche de mesure (10) qui est aménagée pour créer le volume de mesure (30) qui jouxte la surface échantillon autour de la matière stratifiée ou de manière adjacente à la matière stratifiée (20) et
la cloche de mesure (10) comportant une fenêtre (13) perméable à l'énergie et l'élément chauffant par infra-rouges étant aménagé pour irradier la surface échantillon (100) à travers la fenêtre (13), **caractérisé en ce que** le système détecteur (15) est placé à l'intérieur de la cloche de mesure (10) et du volume de mesure (30).

2. Dispositif selon la revendication 1, le dispositif de chauffage étant aménagé pour irradier simultanément une surface partielle de la surface échantillon, la surface partielle ayant une dimension d'au moins 100 cm², de manière plus préférentielle, d'au moins 625 cm² ou même d'au moins 900 cm².

3. Dispositif selon la revendication 1 ou 2, le système détecteur (15) étant aménagé pour quantifier l'au moins un contaminant désorbé hors de la matière stratifiée.

4. Dispositif selon l'une quelconque des revendications précédentes, qui est une partie d'un appareil mobile portable.

5. Dispositif selon la revendication 1, qui comprend par ailleurs un dispositif de rinçage (14a, 14b) qui est aménagé pour rincer au gaz le volume de mesure.

6. Dispositif selon l'une quelconque des revendications précédentes, la matière stratifiée qui doit être examinée étant une matière composite à matrice fibreuse non durcie et/ou une matière composite durcie.

7. Procédé, destiné à examiner une matière stratifiée (20) au niveau des impuretés, qui comprend un échauffement d'une surface échantillon (100) de la matière stratifiée et une détection d'au moins un contaminant (22) désorbé hors de la matière stratifiée dans un volume de mesure (30),
l'échauffement s'effectuant à l'aide d'un élément chauffant par infra-rouges, et
la surface échantillon (100) étant chauffée sous une cloche de mesure (10) qui forme le volume de mesure (30) autour de la matière stratifiée ou de manière adjacente à la matière stratifiée et la cloche de mesure (10) comportant une fenêtre (13) perméable à l'énergie et l'élément chauffant à infra-rouges irradiant la surface échantillon (100) à travers la fenêtre (13), le système détecteur (15) étant placé à l'intérieur de la cloche de mesure (10) et du volume de mesure (30).

8. Procédé selon la revendication 7, l'échauffement comprenant une irradiation simultanée d'une surface partielle de la surface échantillon (100), la surface partielle ayant une dimension d'au moins 100 cm², de manière plus préférentielle, d'au moins 625 cm² ou même d'au moins 900 cm².

9. Procédé selon la revendication 7 ou 8, qui comprend une quantification de l'au moins un contaminant (22) désorbé hors de la matière stratifiée.

10. Procédé selon l'une quelconque des revendications 7 à 9, qui comprend par ailleurs un rinçage au gaz du volume de mesure (30).
